(19) Europäisches Patentamt / European Patent Office / Office européen des brevets

(11) EP 2 050 438 B1

(12) **EUROPEAN PATENT SPECIFICATION**

(45) Date of publication and mention
of the grant of the patent:
**19.10.2016 Bulletin 2016/42**

(51) Int Cl.:
*A61K 9/16* *(2006.01)*    *A61K 9/50* *(2006.01)*
*A61K 31/4152* *(2006.01)*    *A61K 47/38* *(2006.01)*
*A61P 29/00* *(2006.01)*

(21) Application number: **07791287.1**

(22) Date of filing: **25.07.2007**

(86) International application number:
**PCT/JP2007/064571**

(87) International publication number:
**WO 2008/013197 (31.01.2008 Gazette 2008/05)**

(54) **SPHERICAL CRUDE GRANULE AND METHOD FOR PRODUCTION THEREOF**

KUGELFÖRMIGES GROBES GRANULAT UND HERSTELLUNGSVERFAHREN DAFÜR

GRANULÉ BRUT SPHÉRIQUE ET SON PROCÉDÉ DE FABRICATION

(84) Designated Contracting States:
**DE FR GB**

(30) Priority: **26.07.2006 JP 2006203415**

(43) Date of publication of application:
**22.04.2009 Bulletin 2009/17**

(73) Proprietor: **ASAHI KASEI KABUSHIKI KAISHA**
**Tokyo 101-8101 (JP)**

(72) Inventors:
• **YAGINUMA, Yoshihito**
**Tokyo 100-8440 (JP)**
• **MATSUMOTO, Rika**
**Tokyo 100-8440 (JP)**

(74) Representative: **Hoffmann Eitle**
**Patent- und Rechtsanwälte PartmbB**
**Arabellastraße 30**
**81925 München (DE)**

(56) References cited:
EP-A2- 1 238 662    WO-A1-02/36168
WO-A2-99/59544    JP-A- 07 173 050
JP-A- 09 165 329    JP-A- 61 213 201
JP-A- 63 301 816    JP-A- 2000 001 429
JP-A- 2000 128 777    US-A- 5 384 130
US-A- 5 997 905

EP 2 050 438 B1

## Description

Technical Field

The present invention relates to a production technology of film-coated granules which are one of the dosage forms of pharmaceutical preparations.

Background Art

Pharmaceutical solid preparations sometimes have sustained release, enteric, or bitterness-masking film coating with a view to reducing side effects of a drug containing in them, reducing the administration frequency, improving the effect of the drug, suppressing a bitter taste, stabilizing the drug, or the like. Granules having a high sphericity are one of the dosage forms suited for film coating thereon. Such granules are called spherical elementary granules.

As a production method of spherical elementary granules, a method of carrying out extrusion granulation using a drug and an excipient as raw materials and then spheronizing the resulting granulated product (extrusion-spheronization process), a method of coating the surface of spherical seed cores with a drug (layering process) (refer to, for example, Patent Document 1 and Patent Document 2), and the like are known.

In the layering method, granules are produced by coating spherical seed cores with a drug-containing coating layer. Specific examples of it include a method of simultaneously supplying drug powders and an aqueous solution of a binder and coating the spherical seed cores therewith; a method of supplying a suspension of drug particles and coating the spherical seed cores therewith; and a method of supplying an aqueous solution of a drug and coating the spherical seed cores therewith.

The layering method is suited as a method for producing spherical elementary granules to be film-coated, because spherical elementary granules having a high sphericity and a narrow particle size distribution can be obtained by using spherical seed cores having a high sphericity and a narrow particle size distribution.

Spherical elementary granules have desirably a uniform shape, because if there is a difference in the granule shape, the thickness of the film formed by film coating may differ between granules. A sustained release film requires especially precise dissolution control so that uniformity in the shape of spherical elementary granules is important.

Ideal spherical elementary granules are perfect sphere and have a monodisperse distribution, thus the particle size distribution and sphericity of seed cores or elementary granules have conventionally been considered (refer to, for example, Patent Document 3 and Patent Document 4). It is however impossible to realize each of perfect sphericity and monodisperse particle-size distribution.

Patent Document 5 discloses an orally disintegrable tablet, which comprises (i) fine granules having an average particle diameter of 400 $\mu$m or less, which fine granules comprise a composition coated by an enteric coating layer, said composition having 10 weight % or more of an acid-labile physiologically active substance and (ii) an additive, and which allegedly has superior disintegrability or dissolution in the oral cavity so that it can be used for treatment or prevention of various diseases, as an orally disintegrable tablet capable of being administered to the aged or children and easily administered without water.

Patent Document 6 discloses pharmacologically inactive spherical seed cores comprising at least 50% by weight of microcrystalline cellulose having an average degree of polymerization of 60 to 375, wherein the spherical seed cores have an average particle size of 100 to 1000 $\mu$m, a tapped bulk density of at least 0.65 g/ml, an aspect ratio of at least 0.7, a water absorption capacity of 0.5 to 1.5 ml/g, and a friability of no more than 1%; spherical granules comprising the spherical seed cores coated with a powdery layer comprising active ingredients and having an outer layer of coating provided on the powdery layer; and a process for the production of spherical granules, comprising the steps of coating the spherical seed cores with powder comprising active ingredients using an aqueous binding solution, spraying an aqueous solution or suspension of a coating agent thereon, and drying the resulting coated granules.

Patent Document 7 discloses a core of predominately microcrystalline cellulose, on which an active drug is layered onto the core via solution coating. The coated particles have a narrower particle size distribution than coated granules provided by other processes. An optional final coating of a pharmaceutically acceptable polymeric coating is provided to provide tastemaking or controlled release, and protection of the drug-layered particles.

Patent Document 8 discloses coated granules using drug granules containing a water soluble drug as an active ingredient at a high density, which is said to be superior in uniform content and stability, and which is said to be capable of providing a pharmaceutical preparation superior in drug release control and having a smaller size than conventional preparations, and a production method of the granules, and further, a pharmaceutical preparation using the drug granules.

Patent Document 1: Japanese Patent Laid-Open No. Sho 63-301816
Patent Document 2: Japanese Patent Laid-Open No. 2000-1429

Patent Document 3: Japanese Patent Laid-Open No. Hei 7-173050
Patent Document 4: Japanese Patent Laid-Open No. Hei 10-139659
Patent Document 5: WO 99/59544 A2
Patent Document 6: US 5 384 130 A
Patent Document 7: US 5 997 905 A
Patent Document 8: EP 1 238 662 A2

Disclosure of the Invention

Problems to be Solved by the Invention

An object of the present invention is to provide spherical elementary granules having physical properties suited for film coating.

Means for Solving the Problems

With a view to overcoming the above-described problem, the present inventors have carried out an extensive investigation on various physical properties of spherical elementary granules. As a result, it has been found that spherical elementary granules having a certain shape are markedly suited for the production of film-coated granules.

More specifically, it has been found that a short/long diameter ratio distribution, as well as the sphericity and particle size distribution of spherical elementary granules, is an important factor for achieving uniform film coating; and even if spherical elementary granules have a rather low sphericity and do not have, in a precise sense, a monodisperse particle-size distribution, uniform film coating can be realized when the spherical elementary granules have an increased short/long diameter ratio distribution coefficient.

The following are the details of the present invention.

[1] Spherical elementary granules comprising a drug and having a short-diameter distribution coefficient of 0.65 or greater, an average short/long diameter ratio of 0.85 or greater, a short/long diameter ratio distribution coefficient of 0.75 or greater, and a compressive strength of 10 MPa or greater.

[2] The spherical elementary granules according to Item 1, wherein the short-diameter distribution coefficient is 0.65 or greater and not greater than 0.80.

[3] The spherical elementary granules according to Item 1 or 2, wherein the average short/long diameter ratio is 0.85 or greater and not greater than 0.90.

[4] The spherical elementary granules according to any one of Items 1 to 3, wherein the compressive strength is 15 MPa or greater.

[5] The spherical elementary granules according to any one of Items 1 to 4, that have an average short diameter of from 50 to 1200 $\mu$m.

[6] The spherical elementary granules according to Item 1, wherein a content of the drug is 0.01 % by mass or greater.

[7] The spherical elementary granules according to any one of Items 1 to 6, each comprising:

a pharmaceutically inert spherical seed core constituting cores satisfying the following requirements (1) to (4); and
a drug-containing layer which coats the surface of the spherical seed core and
comprises a drug and a water-soluble high-molecular compound:

(1) comprising 30 % by mass or greater of crystalline cellulose,
(2) having an average short diameter of from 50 to 1000 $\mu$m,
(3) having a short-diameter distribution coefficient of 0.60 or greater, an average short/long diameter ratio of 0.80 or greater, and a short/long diameter ratio distribution coefficient of 0.70 or greater, and
(4) having a compressive strength of 10 MPa or greater.

[8] The spherical elementary granules according to Item 7, wherein the spherical seed cores have the compressive strength of 15 MPa or greater.

[9] The spherical elementary granules according to any one of Items 7 to 8, wherein the spherical seed cores have a water absorbing capacity of 0.5 g/cm$^3$ or greater.

[10] The spherical elementary granules according to any one of Items 1 to 9 for the production of film-coated granules.

[11] Film-coated granules comprising the spherical elementary granules as claimed in any one of Items 1 to 9 and a film coating layer coating the surface of the spherical elementary granules.

[12] Use of the spherical elementary granules as claimed in any one of Items 1 to 9 for the production of film-coated granules.

[13] A production method of film-coated granules comprising the step of: coating the spherical elementary granules as claimed in any one of Items 1 to 9 with film.

[14] The production method of spherical elementary granules according to Item 7 comprising the steps of:

spraying an aqueous solution or aqueous suspension comprising a drug and a water-soluble high-molecular compound to the pharmaceutically inert spherical seed cores satisfying the above-described requirements (1) to(4) by using a fluidized-bed film coating apparatus; and

coating the resulting spherical seed cores with the drug-containing layer.

[15] The production method of spherical elementary granules according to Item 14, wherein the fluidized-bed film coating apparatus is either a spouted bed type having, inside thereof, a guide tube (Wurster column) or a fluidized bed type having, on the bottom thereof, a rotating equipment.

Advantage of the Invention

The present invention makes it possible to produce, with high productivity, film-coated granules realizing precise dissolution control.

Best Mode for Carrying Out the Invention

The present invention will hereinafter be described more specifically.

The spherical elementary granules of the present invention are required to have a "spherical" and "uniform" shape.

The term "short-diameter distribution coefficient" of the spherical elementary granules as used herein means a value represented by the following equation:

$$\text{Short-diameter distribution coefficient} = D_{10}/D_{90}$$

wherein, $D_{10}$ and $D_{90}$ represent values at which the cumulative undersize distribution of short diameters reaches 10% and 90%, respectively.

When the short-diameter distribution coefficient of the spherical elementary granules is 0.65 or greater, uniform film coating can be realized even if they are somewhat inferior in sphericity. It is preferably 0.7 or greater, more preferably 0.8 or greater. The theoretical maximum value of the short-diameter distribution coefficient is 1 and it is preferably as close to 1 as possible in order to provide a uniform film coating. Spherical elementary granules having a short-diameter distribution coefficient of 1 are however hardly produced by the existing production technology and production of such granules leads to a marked reduction in the production yield. The maximum value is therefore approximately 0.9 from the viewpoint of production efficiency.

The term "average short/long diameter ratio" of the spherical elementary granules as used herein means a value determined by the following equation:

$$\text{Average short/long diameter ratio} = [D/L]_{50}$$

wherein, $[D/L]_{50}$ represents a value at which the cumulative distribution of the short/long diameter ratios cumulating from the minimum value side reaches 50%.

The average short/long diameter ratio is 0.85 or greater, preferably 0.9 or greater, more preferably 0.95 or greater. The theoretical maximum value of the average short/long diameter ratio is 1, and it is preferably as close to 1 as possible from the viewpoint of providing uniform film coating.

The term "short/long diameter ratio distribution coefficient" of the spherical elementary granules as used herein means a value represented by the following equation:

$$\text{Short/long diameter ratio distribution coefficient} = [D/L]_{10}/[D/L]_{90}$$

wherein, $[D/L]_{10}$ and $[D/L]_{90}$ represent values at which the cumulative distribution of the short/long diameter ratios of the granules cumulating from the minimum value side reaches 10% and 90%, respectively.

The short/long diameter ratio distribution coefficient is 0.7 or greater, preferably 0.8 or greater, more preferably 0.9 or greater. The theoretical maximum value of the coefficient is 1, and it is preferably as close to 1 as possible.

By setting the short/long diameter ratio distribution coefficient at 0.75 or greater, precise sustained-release film coating can be provided and bitter-taste masking with a minimum amount of the film can be achieved even when the short-diameter distribution coefficient and sphericity (average short/long diameter ratio) are as low as from about 0.65 to 0.8 and from about 0.85 to 0.9, respectively.

With regard to the size of the spherical elementary granules, the average short diameter ($D_{50}$) is preferably from about 50 to 1200 $\mu$m.

The term "average short diameter ($D_{50}$)" as used herein means a particle diameter at which the cumulative undersize distribution of short diameters reaches 50%.

The spherical elementary granules of the present invention comprise preferably at least 0.01 % by mass of a drug.

It should be noted that the term "drug" as used herein means what is used for treatment, prevention, or diagnosis of human or animal diseases but what is not an instrument/machine.

Specific examples include anti-epileptic agents (such as phenytoin, acetylpheneturide, trimethadione, phenobarbital, primidone, nitrazepam, sodium valproate, and sultiame), antipyretic, analgesic and anti-inflammatory agents (such as acetaminophen, phenyl acetylglycine methyl amide, mefenamic acid, diclofenac sodium, floctafenine, aspirin, aspirin aluminum, ethenzamide, oxyphenbutazone, sulpyrin, phenylbutazone, ibuprofen, alclofenac, naproxen, ketoprofen, tinoridine hydrochloride, benzydamine hydrochloride, tialamide hydrochloride, indomethacin, piroxicam, and salicylamide), antivertigo agents (such as dimenhydrinate, meclizine hydrochloride, and difenidol hydrochloride), narcotics (such as opium alkaloids hydrochlorides, morphine hydrochloride, codeine phosphate, dihydrocodeine phosphate, and oxymethebanol), agents for psychological use (such as chlorpromazine hydrochloride, levomepromazine maleate, perazine maleate, propericiazine, perphenazine, chlorprothixene, haloperidol, diazepam, oxazepam, oxazolam, mexazolam, alprazolam, and zotepine), skeletal muscle relaxants (such as chlorzoxazone, chlorphenesin carbamate, chlormezanone, pridinol mesylate, and eperisone hydrochloride), autonomic nerve agents (such as betanecol hydrochloride, neostigmine bromide, and pyridostigmine bromide), antispasmodic agents (such as atropine sulfate, butropium bromide, butylscopolamine bromide, propantheline bromide, and papaverine hydrochloride), antiparkinsonian agents (such as biperiden hydrochloride, trihexyphenidyl hydrochloride, amantadine hydrochloride, and levodopa), antihistaminic agents (such as diphenhydramine hydrochloride, dl-chlorpheniramine maleate, promethazine, mequitazine, and clemastine fumarate), cardiotonic agents (such as aminophylline, caffeine, dl-isoproterenol hydrochloride, etilefrin hydrochloride, norfenefrine hydrochloride, and ubidecarenone), antiarrhythmic agents (such as procainamide hydrochloride, pindolol, metoprolol tartrate, and disopyramide), diuretics (such as potassium chloride, cyclopenthiazide, hydrochlorothiazide, triamterene, acetazolamide, and furosemide), antihypertensive agents (such as hexamethonium bromide, hydralazine hydrochloride, syrosingopine, reserpine, propranolol hydrochloride, captopril, and methyldopa), vasoconstrictor agents (such as dihydroergotamine mesylate), vasodilatory agents (such as etafenone hydrochloride, diltiazem hydrochloride, carbochromen hydrochloride, pentaerythritol tetranitrate, dipyridamole, isosorbide nitrate, nifedipine, nicametate citrate, cyclandelate, and cinnarizine), agents for arteriosclerosis (such as ethyl linoleate, lecithin, and clofibrate), agents for the circulatory organs (such as nicardipine hydrochloride, meclofenoxate hydrochloride, cytochrome C, pyridinol carbamate, vinpocetine, calcium hopantenate, pentoxifylline, and idebenone), respiratory stimulants (such as dimefline hydrochloride), antitussives and expectorants (such as dextromethorphan hydrobromide, noscapine, methyl L-cysteine hydrochloride, bromhexine hydrochloride, theophylline, ephedrine hydrochloride, and amlexanox), cholagogues (such as osalmid, phenyl propanol, and hymecromone), agents for intestinal disorders (such as berberine hydrochloride, and loperamide hydrochloride), agents for digestive organs (such as metoclopramide, fenipentol, and domperidone), vitamin preparations (such as retinol acetate, dihydrotachysterol, etretinate, thiamine hydrochloride, thiamine nitrate, fursultiamine, octotiamine, cycotiamine, riboflavin, pyridoxine hydrochloride, pyridoxal phosphate, nicotinic acid, pantethine, cyanocobalamin, biotin, ascorbic acid, phytonadione, and menatetrenone), antibiotics (such as benzathine benzylpenicillin, amoxicillin, ampicillin, cyclacillin, cefaclor, cephalexin, cefuroxime axetil, erythromycin, kitasamycin, josamycin, chloramphenicol, tetracycline, griseofulvin, and cefuzonam sodium), and chemical therapeutic agents (such as sulfamethoxazole, isoniazid, ethionamide, thiazosulfone, nitrofurantoin, enoxacin, ofloxacin, and norfloxacin).

A description will next be made of film coating to be applied to the spherical elementary granules.

In the present invention, spherical elementary granules can be subjected to film coating in order to control a dissolution rate (sustained release, enteric release, timed release, pulse release, bitter-taste masking), moisture prevention or coloring of the drug.

Film coating of the spherical elementary granules can be carried out using a known apparatus such as centrifugal fluidizing coating apparatus ("CF granulator", product of Freund Corporation) or a fluidized-bed coating apparatus. As the fluidized-bed coating apparatus, a spouted bed type having, inside thereof, a guide tube (Wurster column) and a fluidized-bed type with, on the bottom thereof, a rotating equipment, as well as a typical fluidized-bed type, are usable.

Specific examples of such apparatuses include "Flow Coater" and "Spiral Flow", products of Freund Corporation, "WST/WSG Series" and "GPCG Series", products of Glatt GmbH, "New Marumerizer", product of Fuji Paudal Co., Ltd., and "multiplex", product of Powrex Corporation.

A film coating liquid is sprayed to the spherical elementary granules. It may be supplied by a method suited for each of apparatuses such as top spray, bottom spray, side spray, and tangential spray. After completion of spraying, the resulting film-coated granules can be dried as are or after controlling the air flow rate or temperature as needed, without taking out the granules from the apparatus.

The film coating liquid includes, for example, a solution that obtained by dissolving a solid film coating agent in an organic solvent, a dispersion liquid that obtained by dispersing both a film coating agent in fine powder form and a plasticizer in water, or a latex type film coating agent in which a plasticizer is added as needed.

Examples of the film coating agent include acrylic resin coating agents such as a dispersion liquid of ethyl acrylate/methyl methacrylate copolymer, aminoalkyl methacrylate copolymer E, aminoalkyl methacrylate copolymer RS, methacrylic acid copolymer L, methacrylic acid copolymer LD, and methacrylic acid copolymer S; cellulose coating agents such as ethyl cellulose, aqueous dispersion of ethyl cellulose, carboxymethylethyl cellulose, cellulose acetate phthalate, hypromellose phthalate, and hydroxypropylmethyl cellulose acetate succinate; and vinyl acetate resin coating agents such as aqueous dispersion of vinyl acetate resin.

These film coating liquids may comprise an additive such as plasticizer, particles of an inorganic substance, and water soluble substances to adjust the film forming property, coating property, stability, and dissolution property of the film coating agents. Use of organic solvents is not preferred from the viewpoint of protection of working environment or natural environment. A water-based solvent is preferred.

Particularly preferred film coating liquids are latex type coating agents such as dispersion liquid of ethyl acrylate/methyl methacrylate copolymer, aqueous dispersion of methacrylic acid copolymer LD, aqueous dispersion of ethyl cellulose, and aqueous dispersion of vinyl acetate resin.

A spouted fluidized-bed type coating apparatus having a Wurster column or a fluidized-bed type coating apparatus with rotating equipment has been popularly used recently from the viewpoint of productivity and also film coating characteristics to smaller elementary granules. These apparatuses are characterized by a strong tumbling power to be applied to the granules. The granules (spherical elementary granules) subjected to film coating must have a high strength enough for achieving stable/mass production. Since a particularly large amount of spherical elementary granules is charged in a production machine, they must stand, in addition to a stirring power of the apparatus, the gravity of the granules themselves.

The spherical elementary granules of the present invention are therefore required to have a compressive strength of 10 MPa or greater, preferably 15 MPa or greater, more preferably 20 MPa or greater. When they have such a compressive strength, stable film coating can be carried out without causing destruction of the elementary granules or separation of a drug layer.

The term "compressive strength" as used herein is a value represented by the following equation:

$$\text{Compressive strength [MPa]} = 0.7 \times P_0 / \{\pi \times (d/2)^2\}$$

wherein, d means a diameter [$\mu$m] of a granule and $P_0$ means a load [N] at which the granule is destroyed.

The spherical elementary granules of the present invention can be produced by a method of spheronizing after extrusion granulation, a method of spheronizing after granulation by high-speed stirring, and a method (layering method) of coating the surface of spherical seed cores with a drug. Production in accordance with the layering method will next be described as one example.

Spherical seed cores preferably used in the layering process have a crystalline cellulose content of 30 % by mass or greater. The particles having a crystalline cellulose content less than 30 % by mass have difficulty in spheronization and have reduced strength. The crystalline cellulose content is more preferably 70 % by mass, still more preferably 100 % by mass.

The term "crystalline cellulose" as used herein means that conforming to the standards of "crystalline cellulose" specified in the Japanese Pharmacopoeia, Fourteenth Edition.

In the present invention, the spherical seed cores are preferably pharmaceutically inert, meaning that they do not contain a drug. The spherical seed cores may comprise, as a component other than the crystalline cellulose, additives ordinarily used in the production of pharmaceuticals.

Examples include excipients such as lactose, sucrose, D-mannitol, corn starch, powdered cellulose, calcium hydrogen phosphate, and calcium carbonate; disintegrants such as low-substituted hydroxypropyl cellulose, carmellose calcium, pregelatinized starch, croscarmellose sodium, crospovidone, and carboxymethyl starch; binders such as hydroxypropyl cellulose, povidone, and xanthan gum; coating agents such as hydroxypropylmethyl cellulose, methacrylic acid copolymer LD, and aqueous dispersion of ethyl cellulose; emulsifiers such as sucrose fatty acid ester, glycerin fatty acid ester, sodium lauryl sulfate, and polysorbate 60; and other additives such as talc, magnesium stearate, magnesium metasilicate aluminate, titanium oxide, light silicic anhydride, crystalline cellulose/carmellose sodium.

Incorporation of water-soluble pharmaceutical additives may accelerate agglomeration of the particles during layering so that they may be added in an amount of 10 % by mass or less, more preferably 5 % by mass or less.

In the present invention, the spherical seed cores have preferably uniformity in shape. The average short diameter (D50) is preferably from 50 to 1000 $\mu$m.

In addition, the spherical seed cores having a short-diameter distribution coefficient of 0.60 or greater, an average short/long diameter ratio of 0.80 or greater, and a short/long diameter ratio distribution coefficient of 0.7 or greater are preferred. By adjusting them within the above-described ranges, the short-diameter distribution coefficient, average short/long diameter ratio, and short/long diameter ratio distribution coefficient of the spherical elementary granules available by layering the spherical seed cores with the drug-containing layer can be readily adjusted to fall within the range specified by the present invention.

The spherical seed cores have preferably a water absorbing capacity of 0.5 cm$^3$/g or greater. It is more preferably 0.7 cm$^3$/g or greater from the viewpoint of considerable suppression of agglomeration. It is still more preferably 0.9 cm$^3$/g or greater. Although there is no upper limitation, excessive high water absorbing capacity is not preferred because spherical seed cores which have swelled with water absorbed therein shrink during drying step after they are coated with a drug-containing layer, leading to a deterioration in the strength of the resulting spherical elementary granules. The maximum water absorbing capacity of the particles which does not cause swelling due to water absorption is approximately 2.0 cm$^3$/g. A bulk density is determined by the balance between strength and water absorbing capacity and is generally from 0.5 to 2.0 cm$^3$/g. In the case of spherical seed cores composed only of crystalline cellulose, it is generally from 0.5 to 1.0 cm$^3$/g.

The term "water absorbing capacity" as used herein means the volume of water which the spherical seed cores can retain therein per unit mass and it is represented by the following equation:

Water absorbing capacity G [cm$^3$/g] = H/W
H: volume [cm$^3$/g] of water which spherical seed cores can retain therein.
W: mass [g] of the spherical seed cores.

Described specifically, it can be determined by adding 30 mL of purified water to 10 g of a sample (in terms of dry weight), leaving the resulting mixture to stand at room temperature for one hour, filtering out the solid through a filter paper, lightly wiping off water attached to the surface of the solid with another filter paper, measuring the mass of the solid, and dividing a difference calculated by subtracting 10g from the mass (water content) by 10.

In layering (coating) the spherical seed cores with the drug-containing layer, a similar apparatus that is employed in film coating can be used in a similar manner. Preferred examples of the apparatus include, similar to those used for film coating, a spouted fluidized-bed coating apparatus having inside thereof a guide tube (Wurster column) and a fluidized-bed type coating apparatus with, on the bottom thereof, a rotating equipment. A difference resides in that a layering liquid such as an aqueous solution or an aqueous suspension of the drug is used instead of the film coating liquid. A solvent used for the layering liquid may be an organic solvent, but a water-based solvent is preferred from the viewpoint of protecting a working environment or natural environment.

The concentration of the drug in the layering liquid varies, depending on the solubility, viscosity and suspensibility of the drug. It is preferably from about 5 to 30 % by mass. The layering liquid may comprise another pharmaceutical additive as needed.

Water-soluble high-molecular compounds (binders) are most effective as such a pharmaceutical additive. It raises the strength of the drug-containing layer. Specific examples of the water-soluble high-molecular compound include hydroxypropyl cellulose, hypromellose (hydroxypropylmethyl cellulose), methyl cellulose, carmellose sodium, $\alpha$-starch, gum arabic powder, carboxyvinyl polymer, povidone (polyvinylpyrrolidone), polyvinyl alcohol, carrageenan, xanthan gum, and pullulan. Of these, hypromellose (substitution type: 2910) and povidone which have both a binding property and instant-dissolution property of the drug are preferred.

Incorporation of crystalline cellulose/carmellose sodium is also effective for improving suspension stability of drug particles in the case when the layering liquid is an aqueous suspension and for preventing separation of the drug-containing layer from the spherical elementary granules.

Instead of using the layering liquid such as an aqueous solution or aqueous suspension of the drug and the water-soluble high-molecular compound, drug powders and an aqueous solution of the water-soluble high-molecular compound can be supplied simultaneously to the spherical seed cores. In this case, additives other than the drug, such as excipient, may be mixed with the drug powders as needed.

The drug-containing layer is formed by spraying such a layering liquid to the spherical seed cores continuously or intermittently and then drying. During spraying, it is preferred to optimize the amount of hot air, rotation speed of the rotating equipment, spraying pressure of a drug spray liquid, and the like to prevent the spray liquid from drying to become powder (dust) before it reaches the core particles. For this purpose, the compressive strength of the spherical seed cores is preferably 10 MPa or greater. When the compressive strength is low, the particles need to be stirred at a low stirring power. Then, the spray rate of the aqueous solution (aqueous suspension) of the drug decreases, which leads to a reduction in the productivity. The compressive strength is more preferably 15 MPa or greater, still more preferably 20 MPa or greater.

After completion of the spraying of the layering liquid, the resulting spherical elementary granules are dried. They can be dried as are or after controlling the air flow rate and temperature as needed, without taking them out from the apparatus.

A coating amount of the drug-containing layer is determined based on the formulation design such as single dosage or size of the preparation. For example, it is generally from about 0.5 to 200 % by mass relative to the spherical seed cores.

In the spherical elementary granules of the present invention, dissolution of the drug contained therein can be controlled precisely by applying sustained-release film coating to them. The spherical elementary granules themselves do not necessarily have a sustained-release property. Rather, the spherical elementary granules themselves preferably do not have a sustained-release property from the viewpoint of preventing an undissolved residue of the drug. The drug-containing layer is therefore composed mainly of the drug and the water-soluble high-molecular compound. The total amount of the drug and the water-soluble high-molecular compound is preferably 80 % by mass or greater, more preferably 90 % by mass or greater, each of the drug-containing layer. Moreover, the drug-containing layer of the spherical elementary granules preferably does not contain an additive which may reduce the dissolution rate of the drug (for example, a water-insoluble high-molecular compound).

Next, one example of a production method of the spherical elementary granules will be described.

(a) Preparation of layering liquid: A layering liquid is prepared by adding a drug and a necessary pharmaceutical additive to water and thoroughly stirring the resulting mixture to dissolve (suspend) the drug and the optional additives.
(b) Heating of spherical seed cores and fluidized-bed coating apparatus: After spherical seed cores are charged in a fluidized-bed coating apparatus, hot air is supplied from the lower portion of the apparatus until the outlet-air temperature reaches a predetermined temperature and the seed cores are fluidized (a rotor portion is rotated simultaneously in the case where a fluidized-bed type coating apparatus with rotating equipment is used as the fluidized-bed coating apparatus).
(c) Coating with drug-containing layer: The layering liquid is sprayed at a predetermined spray rate continuously or intermittently or at a rate raised in a stepwise fashion. The supply of the layering liquid is terminated when the coating amount reaches a predetermined amount.
(d) Drying of spherical elementary granules: The spherical elementary granules are dried while adjusting the amount of hot air and temperature (rotation speed of the rotor portion in the case a fluidized-bed type coating apparatus with rotating equipment is employed) if necessary.
(e) Taking-out of spherical elementary particles: In the end, the resulting spherical elementary particles are taken out.

The spherical elementary granules obtained by the above process can be used as granules, capsules, tablets or the like after subjected to particle size regulation and sustained release film coating, enteric film coating, or bitterness masking film coating if necessary.

Examples

The present invention will next be described based on some examples. First, the measuring methods of physical properties will be described below collectively.

<Average short diameter, short-diameter distribution coefficient, short/long diameter ratio distribution coefficient, and average short/long diameter ratio of spherical seed cores and spherical elementary granules>

The shape of a sample is photographed using a digital microscope ("VH-7000", product of KEYENCE COR-PORATION) (with a $50\times$ or $100\times$ lens) and a short diameter (D) and a long diameter (L) of 100 particles are measured using an image analyzer ("Image Hyper", product of Inter Quest). The terms "short diameter" and "long diameter" as used herein mean the length of a short side and the length of a long side of the smallest rectangle that circumscribes boundary pixels of a particle, respectively.

The particle sizes at which the cumulative undersize distribution of short diameters reach 10%, 50%, and 90% are represented by "$D_{10}$", "$D_{50}$", and "$D_{90}$", respectively. The average short diameter means $D_{50}$, and the short-diameter distribution coefficient means $D_{10}/D_{90}$.

A ratio of the short diameter to the long diameter (short/long diameter ratio) is represented by D/L and short/long diameter ratios at which the cumulative distribution of the short/long diameter ratios cumulating from the minimum value side reach 10%, 50%, and 90% are represented by "$[D/L]_{10}$", "$[D/L]_{50}$", and "$[D/L]_{90}$", respectively. The short/long diameter ratio distribution coefficient means $[D/L]_{10}/[D/L]_{90}$, and the average short/long diameter ratio means $[D/L]_{50}$.

$$\text{Average short diameter } [\mu m] = D_{50}$$

$$\text{Short-diameter distribution coefficient } [-] = D_{10}/D_{90}$$

$$\text{Short/long diameter ratio distribution coefficient } [-] = [D/L]_{10}/[D/L]_{90}$$

$$\text{Average short/long diameter ratio } [-] = [D/L]_{50}$$

compressive strength [MPa] of spherical seed cores and spherical elementary granules>

The diameter (d [$\mu$m]) and breaking load (P[N]) of particle or granule are measured using a granule strength measuring apparatus ("GRANO", product of Okada Seiko Co., Ltd.). A load cell having a rated capacity of 5N or 20N is used. A chip is displaced down at a rate of 100 $\mu$m/s in order to apply a load to the particle or granule. A change of a load that the chip is subject to by the particle or granule with respect to the displacement of the chip is graphed. A point at which the load decreases by 0.15N or greater is determined as a breaking point and the load applied to the particle or granule at this time is determined as a breaking load $P_0$[N]. The compressive strength is calculated in accordance with the following equation. The above measurement is performed for 50 particles or granules, and average of them is calculated.

$$\text{Compressive strength } [MPa] = 0.7 \times P_0/\{\pi \times (d/2)^2\}$$

<collection ratio [% by mass] of spherical elementary granules and film-coated granules>

The collection ratio is determined in accordance with the following equation based on the collection amount [g] of spherical elementary granules or film-coated granules and the total amount [g] of raw materials employed.

$$\text{Collection ratio } [\% \text{ by mass}] = \{\text{collection amount } [g]/\text{total amount } [g] \text{ of raw materials}\} \times 100$$

<Agglomeration ratio [%] of spherical elementary granules and film-coated granules>

After dispersion of spherical elementary granules or film-coated granules on paper, the number of particles [a] constituting agglomerated granules and the number of single isolated particles [b] are counted visually. The agglomeration ratio is calculated in accordance with the following equation. The number of particles observed is 1000 (=a+b).

$$\text{Agglomeration ratio } [\%] = \{a/(a+b)\} \times 100$$

<Bitter-taste perception time [sec] of film-coated granules>

A panel of three experts put 0.5 g of film-coated granules into their mouth and placed them on their tongue. An average of the time required to perceive a bitter taste without moving the tongue is determined as a bitter-taste detection time.

[Example 1]

(Production of seed cores)

Crystalline cellulose (10 kg) having an average polymerization degree of 220 was charged in a tumbling fluidized-bed granulator ("Multiplex MP-25", product of Powrex Corporation) and 14 kg of distilled water was sprayed in the top spray manner at a rate of 100 g/min under the conditions of a rotation speed of 336 ppm, an air flow rate of from 1.7 to 4.5 $m^3$/min, and an inlet-air temperature of 55°C. Without changing the conditions, tumbling and flowing were performed for 60 minutes. Then, the inlet-air temperature was changed to 70°C, the rotation speed was decreased by 50 rpm every 20 minutes, and drying was performed until the outlet-air temperature became 35°C. After drying, coarse particles having a size of 710 μm or greater and fine powders having a size of 300 μm or less were screened out to obtain spherical seed cores A.

(Production of spherical elementary granules)

While stirring 102 g of water with propeller, 3 g of povidone ("K-30", product of ISP Tec. Inc.) and 15 g of sulpyrine (product of Merck Hoei, Ltd.) were charged therein and the mixture was stirred until completely dissolved to obtain a layering liquid. In a spouted type (Wurster type) coating apparatus ("Multiplex MP-01" using a Wurster column, product of Powrex Corporation), 0.5 kg of the above mentioned spherical seed cores were charged, followed by preliminary heating under the conditions of a spray air pressure of 0.16 MPa, a spray air flow rate of 40 L/min, a charge-air temperature of 75°C, and an air flow rate of from 31 to 43 $m^3$/h until an outlet-air temperature became 40°C. Layering was performed under the conditions of a layering liquid spray rate of 3 g/min (corresponding to a coating rate of 0.9 g/min in solid content per kg of spherical seed cores) until a coating amount for the spherical seed cores became 2.4 % by mass (2.0 % by mass in terms of the drug). The above operation was performed twice and the granules thus layered were combined to obtain spherical elementary granules.
The spherical elementary granules thus obtained have a high collection ratio and a low agglomeration ratio. The results are shown in Table 1.

(Film coating)

For film coating to mask a bitter taste, a film coating liquid consisting of 10.9 parts (solid content) of an aqueous dispersion of ethyl cellulose ("Aquacoat, ECD-30", solid concentration: 30 % by mass, product of FMC Corporation), 2.7 parts of triethyl citrate (product of Tokyo Kasei Kogyo), 1.4 parts of D-mannitol (product of Towa Kasei Kogyo), and 85 parts of water was prepared. The spherical elementary granules (0.8 kg) were charged in a tumbling fluidized-bed coating apparatus ("Multiplex MP-01 ", product of Powrex Corporation) and pre-heated while being tumbled and fluidized under the conditions of an inlet-air temperature of 75°C, an air flow rate of from 37 to 50 $m^3$/h, and a rotation speed of a rotor of 200 rpm until an outlet-air temperature became 38°C. The spherical elementary granules were then coated using a tangential bottom spray until the solid content which derived from the film coating liquid reached 15 % by mass under the conditions of a spray air pressure of 0.16 MPa, a spray air flow rate of 40 L/min, an outlet-air temperature of from 36 to 38°C, and a spray rate of coating liquid of 10.0 g/min. After completion of the coating, the granules were heated until the inlet-air temperature became 40°C at a rotation speed of a rotor at 200 rpm. Then, the heater was turned off and the granules were cooled until the inlet-air temperature became 40°C. The granules thus obtained were spread widely over a tray and cured (heat treated to form a film) for 60 minutes in an oven of 80°C to obtain film-coated granules.
A bitter taste of the film-coated granules thus obtained was masked for about 48 seconds and the film-coated granules had a very low agglomeration. The results are shown in Table 1.

[Example 2]

(Production of seed cores)

Crystalline cellulose (10 kg) having an average polymerization degree of 220 was charged in a tumbling fluidized-bed granulator ("Multiplex MP-25", product of Powrex Corporation) and 14 kg of distilled water was sprayed in the top spray manner at a rate of 200 g/min under the conditions of a rotation speed of 336 rpm, an air flow rate of from 1.7 to 4.5 $m^3$/min, and an inlet-air temperature of 55°C. Without changing the conditions, tumbling and fluidization were performed for 60 minutes. The inlet-air temperature was then raised to 80°C. The rotation speed was reduced by 50 rpm every 20 minutes and drying was performed until the outlet-air temperature became 35°C. After drying, coarse particles having a size of 710 μm or greater and fine powders having a size of 300 μm or less were screened out to obtain spherical seed cores B.

The physical properties of the seed cores are shown in Table 2.

(Production of spherical elementary granules)

In the same manner as Example 1, layering was performed to obtain spherical elementary granules.
The spherical elementary granules corresponding to almost all of the amount of the charged raw materials were collected, and in addition they contained only a small amount of agglomerated granules. The results are shown in Table 1.

(Film Coating)

In the same manner to Example 1, film coating was performed to obtain film-coated granules.
A bitter taste from the film-coated granules thus obtained was masked for about 29 seconds, and in addition the film-coated granules had a low agglomeration. The results are shown in Table 1.

[Example 3]

(Production of spherical elementary granules)

While stirring 540 g of water with propeller, 10 g of povidone ("K-30", product of ISP Tec. Inc.) and 50 g of sulpyrine (product of Merck Hoei, Ltd.) were charged therein and the mixture was stirred until completely dissolved to obtain a layering solution was prepared. In a tumbling type fluidized bed coating apparatus ("multiplex MP-01", product of Powrex Corporation), 1.0 kg of the spherical seed cores A obtained in Example 1 were charged, and pre-heated under the conditions of an inlet-air temperature of 75°C, an air flow rate of from 37 to 50 m$^3$/h and a rotation speed of a rotor of 200 rpm until an outlet-air temperature became 40°C. Layering was performed under the conditions of a rotation speed of a rotor of 380 rpm, a spray air pressure of 0.16 MPa, a spray air flow rate of 40 L/min, an inlet-air temperature of 75°C, an outlet-air temperature of 40°C, an air flow rate of from 37 to 50 m$^3$/h, and a layering liquid spray rate of 8.0 g/min (corresponding to a coating rate of 0.8 g/min in solid content per kg of spherical seed cores) until the coating amount for the spherical seed cores became 6.0 % by mass (5.0 % by mass in terms of the drug). Then, the rotation speed of a rotor was reduced to 200 rpm and the resulting particles were dried until the outlet-air temperature increased to 42°C. A heater for inlet-air was turned off and the inlet-air was cooled to 40°C.
An amount of the resulting spherical elementary granules which attached to the inner wall of the coating apparatus was small and almost all the amount of them was collected. In addition, they were substantially free from agglomeration. The results are shown in Table 1.

(Film coating)

In the same manner as Example 1, film coating was performed to obtain film-coated granules.
A bitter taste of the film-coated granules thus obtained was masked for about 35 seconds, and in addition the film-coated granules had a low agglomeration. The results are shown in Table 1.

[Example 4]

(Production of seed cores)

Crystalline cellulose (10 kg) having an average polymerization degree of 140 was charged in a tumbling fluidized-bed granulator ("Multiplex MP-25", product of Powrex Corporation) and 11 kg of distilled water was sprayed in the top spray manner at a rate of 150 g/min under the conditions of a rotation speed of 250 rpm, an air flow rate of from 3.5 to 4.5 m$^3$/min, and an inlet-air temperature of 50°C. Without changing the conditions, tumbling and fluidization were performed for 30 minutes. Then, the inlet-air temperature was raised to 80°C, the rotation speed was reduced by 50 rpm every 20 minutes, and drying was performed until the outlet-air temperature became 35°C. After drying, coarse particles having a size of 710 μm or greater and fine powders having a size of 300 μm or less were screened out to obtain spherical seed cores C.
The physical properties of the spherical seed cores are shown in Table 2.

(Production of spherical elementary granules)

In the same manner as Example 1, layering was performed to obtain spherical elementary granules.

The spherical elementary granules corresponding to almost all of the amount of the charged raw materials were collected, and in addition the film-coated granules had a low agglomeration. The results are shown in Table 1.

(Film coating)

In the same manner as Example 1, film coating was performed to obtain film-coated granules.

A bitter taste from the film-coated granules thus obtained was masked for about 53 seconds and in addition, the film-coated granules had a low agglomeration. The results are shown in Table 1.

[Example 5]

(Production of spherical elementary granules)

In the same manner as Example 3 except that the spherical seed cores C obtained in Example 4 was used as the spherical seed cores , layering was performed to obtain spherical elementary granules.

The spherical elementary granules corresponding to almost all of the charged raw materials were collected, and in addition the film-coated granules had a low agglomeration. The results are shown in Table 1.

(Film Coating)

In the same manner as Example 1, film coating was performed to obtain film-coated granules.

The film-coated granules thus obtained were delayed in releasing their bitter taste by about 51 seconds, and in addition the film-coated granules had a low agglomeration. The results are shown in Table 1.

[Comparative Example 1]

(Production of seed cores)

Crystalline cellulose (10 kg) having an average polymerization degree of 220 were charged in a tumbling fluidized-bed granulator ("Multiplex MP-25", product of Powrex Corporation) and 14 kg of distilled water was sprayed in the top spray manner at a rate of 200 g/min under the conditions of a rotation speed of 250 rpm, an air flow rate of from 3.5 to 5.5 $m^3$/min, and an inlet-air temperature of 55°C. Then, the air flow rate was raised to 8 $m^3$/min, the inlet-air temperature was raised to 80°C, the rotation speed was reduced by 50 rpm every 20 minutes, and drying was performed until the outlet-air temperature became 35°C. After drying, coarse particles having a size of 500 $\mu$m or greater and fine powders having a size of 250 $\mu$m or less were screened out to obtain spherical seed cores (a).

The physical properties of the seed cores are shown in Table 2.

(Production of spherical elementary granules)

In the same manner to Example 1, layering was performed to obtain spherical elementary granules.

The spherical elementary granules corresponding to almost all of the charged raw materials was collected, but they contained a large amount of agglomerated granules. The results are shown in Table 1.

(Film coating)

In the same manner as Example 1, film coating was performed to obtain film-coated granules.

A bitter taste of the resulting film-coated granules was perceived about 8 seconds after administration and thus, their bitter taste was not masked sufficiently. The results are shown in Table 1.

[Comparative Example 2]

(Production of spherical elementary granules)

In the same manner as Example 3 except for the use of, as the spherical seed cores, the spherical seed cores (a) obtained in Comparative Example 1, layering was performed to obtain spherical elementary granules.

Almost all the amount of the spherical elementary granules was collected but they contained a large amount of agglomerated granules. The results are shown in Table 1.

(Film coating)

In the same manner as Example 1, film coating was performed to obtain film-coated granules.
A bitter taste of the resulting film-coated granules was perceived about 11 seconds after administration and thus, their bitter taste was not masked sufficiently. The results are shown in Table 1.

[Comparative Example 3]

(Production of spherical elementary granules)

Crystalline cellulose (200 g) having an average polymerization degree of 140, 132.2 g of lactose ("Pharmatose 200M", product of DMV), 60 g of corn starch (product of Nippon Starch Chemical Co., Ltd.), and 7.8 g of sulpyrine were charged in a planetary mixer ("5DM-03-R", beater type paddle, product of Shinagawa Seisakujo Co., Ltd.) and stirred at 63 rpm. To the resulting mixture was added 240 g of water and they were mixed as were for 5 minutes. The mixture thus obtained was granulated in an extrusion granulator ("Dome Gran Model DG-L1", a die having a pore size of 300 μm, screw rotation speed: 40 rpm, product of Fuji Paudal Co., Ltd.), followed by spheronization at 690 rpm for 20 minutes in a spheronizing equipment ("Marumerizer Q-230 Type", product of Fuji Paudal Co., Ltd.). The above-described operation was performed three times and granulated products thus obtained were combined and dried at 45°C for 16 hours in an oven. Coarse particles having a size of 710 μm or greater and fine powders having a size of 300 μm or less were screened out to obtain spherical elementary granules containing 1.95 % by mass of sulpyrine. The physical properties of the granules are shown in Table 1.

(Film Coating)

In the same manner as Example 1, film coating was performed to obtain film-coated granules.
A bitter taste of the granules was perceived about 9 seconds after administration. Thus, a bitter taste was not masked sufficiently. The results are shown in Table 1.

[Comparative Example 4]

(Production of spherical elementary granules)

In the same manner as Comparative Example 3 except that the amount of water added was changed to 280 g, granulation was performed to obtain spherical elementary granules comprising 1.95 % by mass of sulpyrine. The physical properties of the granules are shown in Table 1.

(Film coating)

In the same manner as Example 1, film coating was performed to obtain film-coated granules.
A bitter taste of the granules was perceived about 13 seconds after administration. Thus, a bitter taste was not masked sufficiently. The results are shown in Table 1.
The results of Examples 1 to 5 and Comparative Examples 1 to 4 are shown in Table 1.
The spherical elementary granules obtained in Examples 1 to 5 having a short/long diameter ratio distribution coefficient, an average short/long diameter ratio, and a short-diameter distribution coefficient each falling within the range specified by the present invention did not agglomerate much during film coating and release of a bitter taste from them was masked sufficiently.
In contrast, the spherical elementary granules obtained in Comparative Examples 1 to 4, agglomerated during film coating and release of a bitter taste from them was not masked sufficiently.
Even though the spherical elementary granules had, as those in Examples 2, 4 and 5, a relatively low average short/long diameter ratio and short-diameter distribution coefficient and were neither perfect sphere nor mono-disperse, agglomeration during film coating was prevented and uniform film coating with sufficient masking of a bitter taste was achieved by adjusting the short/long diameter ratio distribution coefficient of the spherical elementary granules to fall within the range specified by the present invention.

[Table 1]

| | Physical properties of spherical elementary granules | | | | | Results of film coating | | | Spherical seed cores used | Results of layering | |
|---|---|---|---|---|---|---|---|---|---|---|---|
| | Short-diameter distribution coefficient H | Average short/long diameter ratio [-] | Short/long diameter-ratio distribution coefficient [-] | Average short diameter [μm] | Compressive strength [MPa] | Collection ratio [%] | Agglomeration ratio [%] | Bitter-taste perception time [sec] | | Collection ratio [%] | Agglomeration ratio [%] |
| Example 1 | 0.85 | 0.91 | 0.84 | 400 | 29.8 | 97.8 | 0.9 | 48 | A | 96.7 | 0.5 |
| Example 2 | 0.79 | 0.85 | 0.77 | 464 | 15.6 | 96.1 | 2.7 | 29 | B | 92.2 | 3.2 |
| Example 3 | 0.85 | 0.91 | 0.83 | 403 | 30.3 | 96.9 | 0.7 | 35 | A | 98.1 | 0.2 |
| Example 4 | 0.65 | 0.89 | 0.76 | 514 | 10.6 | 98.2 | 1.3 | 53 | C | 96.3 | 3.3 |
| Example 5 | 0.69 | 0.90 | 0.75 | 518 | 10.8 | 97.9 | 1.5 | 51 | C | 96.8 | 3.6 |
| Comp. Ex. 1 | 0.66 | 0.87 | 0.70 | 323 | 9.4 | 95.4 | 8.8 | 8 | a | 91.8 | 9.6 |
| Comp. Ex. 2 | 0.71 | 0.88 | 0.68 | 315 | 9.0 | 94.7 | 7.9 | 11 | a | 81.0 | 4.6 |
| Comp. Ex. 3 | 0.60 | 0.90 | 0.79 | 461 | 8.5 | 95.1 | 4.3 | 9 | - | - | - |
| Comp. Ex. 4 | 0.72 | 0.81 | 0.81 | 475 | 11.3 | 94.9 | 3.8 | 13 | - | - | - |

EP 2 050 438 B1

[Table 2]

| Spherical seed cores | Short-diameter distribution coefficient [-] | Average short/long diameter ratio [-] | Short/long diameter-ratio distribution coefficient H | Average short diameter [μm] | Compressive strength [MPa] | Content of crystalline cellulose [%] |
|---|---|---|---|---|---|---|
| A | 0.83 | 0.90 | 0.84 | 397 | 28.0 | 100 |
| B | 0.71 | 0.84 | 0.71 | 462 | 14.3 | 100 |
| C | 0.64 | 0.89 | 0.75 | 511 | 10.4 | 100 |
| A | 0.63 | 0.85 | 0.68 | 313 | 9.0 | 100 |

[Example 6]

Sustained-release film-coated granules were prepared using the spherical elementary granules obtained in Example 2.

Described specifically, a film coating liquid composed of 11.5 parts (solid content) of an aqueous dispersion of ethyl cellulose ("Celioscoat EC-30A" having a solid concentration of 30 % by mass, product of Asahi Kasei Chemicals Corporation), 2.9 parts of triethyl citrate (product of Tokyo Chemical industry Co., Ltd.), 0.6 part of D-mannitol (product of Towa Kasei Kogyo Co., Ltd.), and 85 parts of water was prepared. Then, 0.5 kg of the spherical elementary granules obtained in Example 2 was charged in a spouted (Wurster type) coating apparatus ("GPCG-1 type", product of Glatt GmbH) and the film coating liquid was applied to the spherical elementary granules under the conditions of an inlet-air temperature of 65°C, an outlet-air temperature of from 47 to 50°C, an air flow rate of 80 m$^3$/h, a spray air pressure of 0.16 MPa, and a coating solution spray rate of 2.0 g/min until the solid content which derived from the film coating liquid became 5 % by mass. In order to prevent commingling of sulpyrine with the film, the coated granules were dried until the outlet-air temperature became 53°C and then coating was performed again. The coating was performed under the same conditions as the above-described ones except that the coating liquid spray rate was changed to from 3.0 to 4.8 g/min. After completion of the coating, the resulting granules were heated until the outlet-air temperature became 53°C. Then, the heater was turned off and the inlet-air temperature was cooled to 36°C. The granules thus obtained were spread widely over a tray and cured (heating-treated for film formation) for 60 minutes in an oven of 80°C to obtain sustained-release film-coated granules.

The dissolution rate of sulpyrine from the resulting film-coated granules was measured in accordance with Method 2 (paddle method) of "Dissolution Test" in general tests of the Japanese Pharmacopoeia, Fourteenth Edition. The rotation speed of a paddle was set at 100 rpm and "Dissolution test 1st fluid" was used as a test fluid. As a result of measurement, dissolution ratios of sulpyrine from the film-coated granules after 2 hours, 4 hours, 6 hours, 8 hours, and 10 hours were 37.5%, 54.1%, 63.2%, 69.1%, and 73.1%, respectively.

[Comparative Example 5]

The spherical elementary granules obtained in the same manner as Comparative Example 3 were film-coated as in Example 6 to obtain sustained-release film-coated granules. The dissolution ratios of sulpyrine from the film-coated granules after 2 hours, 4 hours, 6 hours, 8 hours, and 10 hours were 41.9%, 60.4%, 70.2%, 76.5%, and 81.0%, respectively.

The dissolution rate of sulpyrine from the film-coated granules obtained in Comparative Example 5 was about 10% greater than that of sulpyrine from the film-coated granules obtained in Example 6. This is presumed to be due to the fact that the short-diameter distribution coefficient of the spherical elementary granules used in Comparative Example 5 is smaller.

Industrial Applicability

The production method of the present invention is suitably used in the field of production of film-coated pharmaceutical granules.

**Claims**

1. Spherical elementary granules comprising a drug and having a short-diameter distribution coefficient of 0.65 or greater, an average short/long diameter ratio of 0.85 or greater, a short/long diameter ratio distribution coefficient of 0.75 or greater, and a compressive strength of 10 MPa or greater.

2. The spherical elementary granules according to Claim 1, wherein the short-diameter distribution coefficient is 0.65 or greater and not greater than 0.80.

3. (Currently amended) The spherical elementary granules according to Claim 1 or 2, wherein the average short/long diameter ratio is 0.85 or greater and not greater than 0.90.

4. The spherical elementary granules according to any one of Claims 1 to 3, wherein the compressive strength is 15 MPa or greater.

5. The spherical elementary granules according to any one of Claims 1 to 4, that have an average short diameter of from 50 to 1200 $\mu$m.

6. The spherical elementary granules according to Claim 1, wherein a content of the drug is 0.01 % by mass or greater.

7. The spherical elementary granules according to any one of Claims 1 to 6, each comprising:

   a pharmaceutically inert spherical seed core constituting cores satisfying the following requirements (1) to (4); and
   a drug-containing layer which coats the surface of the spherical seed core and
   comprises a drug and a water-soluble high-molecular compound:

      (1) comprising 30 % by mass or greater of crystalline cellulose,
      (2) having an average short diameter of from 50 to 1000 $\mu$m,
      (3) having a short-diameter distribution coefficient of 0.60 or greater, an average short/long diameter ratio of 0.80 or greater, and a short/long diameter ratio distribution coefficient of 0.70 or greater, and
      (4) having a compressive strength of 10 MPa or greater.

8. The spherical elementary granules according to Claim 7, wherein the spherical seed cores have the compressive strength of 15 MPa or greater.

9. The spherical elementary granules according to any one of Claims 7 to 8, wherein the spherical seed cores have a water absorbing capacity of 0.5 g/cm$^3$ or greater.

10. The spherical elementary granules according to any one of Claims 1 to 9 for the production af film-coated granules.

11. Film-coated granules comprising the spherical elementary granules as claimed in any one of Claims 1 to 9 and a film coating layer coating the surface of the spherical elementary granules.

12. Use of the spherical elementary granules as claimed in any one of Claims 1 to 9 for the production of film-coated granules.

13. A production method of film-coated granules comprising the step of:

   coating the spherical elementary granules as claimed in any one of Claims 1 to 9 with film.

14. The production method of spherical elementary granules according to Claim 7 comprising the steps of:

   spraying an aqueous solution or aqueous suspension comprising a drug and a water-soluble high-molecular compound to the pharmaceutically inert spherical seed cores satisfying the above-described requirements (1) to(4) by using a fluidized-bed film coating apparatus; and
   coating the resulting spherical seed cores with the drug-containing layer.

15. The production method of spherical elementary granules according to Claim 14, wherein the fluidized-bed film

# EP 2 050 438 B1

coating apparatus is either a spouted bed type having, inside thereof, a guide tube (Wurster column) or a fluidized bed type having, on the bottom thereof, a rotating equipment.

**Patentansprüche**

1. Sphärische elementare Körnchen, umfassend ein Arzneimittel und mit einem Verteilungskoeffizienten des kurzen Durchmessers von 0,65 oder mehr, einem durchschnittlichen kurzen/langen Durchmesserverhältnis von 0,85 oder mehr, einem kurzen/langen Durchmesserverhältnis-Verteilungskoeffizienten von 0,75 oder mehr und einer Kompressionsfestigkeit von 10 MPa oder mehr.

2. Sphärische elementare Körnchen gemäß Anspruch 1, worin der kurze Durchmesserverteilungskoeffizient 0,65 oder mehr und nicht mehr als 0,80 ist.

3. Sphärische elementare Körnchen gemäß Anspruch 1 oder 2, worin das durchschnittliche kurze/lange Durchmesserverhältnis 0,85 oder mehr und nicht mehr als 0,90 ist.

4. Sphärische elementare Körnchen gemäß einem der Ansprüche 1 bis 3, worin die Kompressionsfestigkeit 15 MPa oder mehr ist.

5. Sphärische elementare Körnchen gemäß einem der Ansprüche 1 bis 4, die einen durchschnittlichen kurzen Durchmesser von 50 bis 1.200 $\mu$m haben.

6. Sphärische elementare Körnchen nach Anspruch 1, worin ein Gehalt des Arzneimittels 0,01 Masse% oder mehr ist.

7. Sphärische elementare Körnchen gemäß einem der Ansprüche 1 bis 6, jeweils umfassend:

   einen pharmazeutisch inerten sphärischen Keimkern, der die Kerne ausmacht, die die folgenden Erfordernisse (1) bis (4) erfüllen, und
   eine arzneimittelhaltige Schicht, die die Oberfläche des sphärischen Keimkerns beschichtet und ein Arzneimittel und eine wasserlösliche hochmolekulare Verbindung enthält:

   (1) umfassend 30 Masse% oder mehr kristalline Cellulose,
   (2) mit einem durchschnittlichen kurzen Durchmesser von 50 bis 1.000 $\mu$m,
   (3) mit einem kurzen Durchmesserverteilungskoeffizienten von 0,60 oder mehr, einem durchschnittlichen kurzen/langen Durchmesserverhältnis von 0,80 oder mehr und einem kurzen/langen Durchmesserverhältnis-Verteilungskoeffizienten von 0,70 oder mehr und
   (4) mit einer Kompressionsfestigkeit von 10 MPa oder mehr.

8. Sphärische elementare Körnchen gemäß Anspruch 7, worin die sphärischen Keimkörner eine Kompressionsfestigkeit von 15 MPa oder mehr haben.

9. Sphärische elementare Körnchen gemäß einem der Ansprüche 7 bis 8, worin die sphärischen Keimkerne eine Wasserabsorptionsfähigkeit von 0,5 g/cm$^3$ oder mehr haben.

10. Sphärische elementare Körnchen gemäß einem der Ansprüche 1 bis 9, zur Erzeugung von filmbeschichteten Körnchen.

11. Filmbeschichtete Körnchen, umfassend die sphärischen elementaren Körnchen gemäß einem der Ansprüche 1 bis 9 und eine Filmbeschichtungsschicht, die die Oberfläche der sphärischen elementaren Körnchen beschichtet.

12. Verwendung der sphärischen elementaren Körnchen gemäß einem der Ansprüche 1 bis 9 zur Erzeugung von filmbeschichteten Körnchen.

13. Produktionsverfahren für filmbeschichtete Körnchen, umfassend den Schritt: Beschichten der sphärischen elementaren Körnchen gemäß einem der Ansprüche 1 bis 9 mit Film.

14. Produktionsverfahren von sphärischen elementaren Körnchen gemäß Anspruch 7, umfassend die Schritte:

Sprühen einer wässrigen Lösung oder wässrigen Suspension, umfassend ein Arzneimittel und eine wasser-löslichte hochmolekulare Verbindung, auf pharmazeutisch inerte sphärische Keimkerne, die die oben beschriebenen Erfordernisse (1) bis (4) erfüllen, durch Verwendung einer Fließbett-Filmbeschichtungsanlage, und Beschichten der resultierenden sphärischen Keimkerne mit der arzneimittelhaltigen Schicht.

15. produktionsverfahren von sphärischen elementraren Körnchen gemäß Anspruch 14, worin die Fließbett-Filmbeschichtungsanlage entweder vom Spritzbetttyp mit einem Führungsrohr (Wurster-Säule) im Inneren davon oder Fließbetttyp mit einer Rotationsanlage am Boden davon ist.

**Revendications**

1. Granulés élémentaires sphériques comprenant un médicament et présentant un coefficient de distribution de diamètres courts supérieur ou égal à 0,65, un rapport moyen diamètre court/diamètre long supérieur ou égal à 0,85, un coefficient de distribution de rapport diamètre court/diamètre long supérieur ou égal à 0,75, et une résistance à la compression supérieure ou égale à 10 MPa.

2. Granulés élémentaires sphériques selon la revendication 1, dans lesquels le coefficient de distribution de diamètres courts est supérieur ou égal à 0,65 et n'est pas supérieur à 0,80.

3. Granulés élémentaires sphériques selon la revendication 1 ou 2, dans lesquels le rapport moyen diamètre court/diamètre long est supérieur ou égal à 0,85 et n'est pas supérieur à 0,90.

4. Granulés élémentaires sphériques selon l'une quelconque des revendications 1 à 3, dans lesquels la résistance à la compression est supérieure ou égale à 15 MPa.

5. Granulés élémentaires sphériques selon l'une quelconque des revendications 1 à 4, qui possèdent un diamètre court moyen de 50 à 1200 $\mu$m.

6. Granulés élémentaires sphériques selon la revendication 1, dans lesquels une teneur du médicament est supérieure ou égale à 0,01 % en masse.

7. Granulés élémentaires sphériques selon l'une quelconque des revendications 1 à 6, chacun comprenant :

   un noyau de semence sphérique pharmaceutiquement inerte constituant des noyaux satisfaisant aux exigences (1) à (4) suivantes ; et
   une couche contenant un médicament qui recouvre la surface du noyau de semence sphérique et qui comprend un médicament et un composé hydrosoluble de masse moléculaire élevée :

   1) comprenant 30 % en masse ou plus de cellulose cristalline,
   (2) présentant un diamètre court moyen de 50 à 1000 $\mu$m,
   (3) présentant un coefficient de distribution de diamètres courts supérieur ou égal à 0,60, un rapport moyen diamètre court/diamètre long supérieur ou égal à 0,80, et un coefficient de distribution de rapport diamètre court/diamètre long supérieur ou égal à 0,70, et
   (4) présentant une résistance à la compression supérieure ou égale à 10 MPa.

8. Granulés élémentaires sphériques selon la revendication 7, dans lesquels les noyaux de semence sphériques présentent une résistance à la compression supérieure ou égale à 15 MPa.

9. Granulés élémentaires sphériques selon l'une quelconque des revendications 7 à 8, dans lesquels les noyaux de semence sphériques présentent une capacité d'absorption d'eau supérieure ou égale à 0,5 g/cm$^3$.

10. Granulés élémentaires sphériques selon l'une quelconque des revendications 1 à 9, pour la production de granulés pelliculés.

11. Granulés pelliculés comprenant les granulés élémentaires sphériques selon l'une quelconque des revendications 1 à 9 et une couche de revêtement pelliculaire recouvrant la surface des granulés élémentaires sphériques.

**12.** Utilisation des granulés élémentaires sphériques selon l'une quelconque des revendications 1 à 9 pour la production de granulés pelliculés.

**13.** Procédé de production de granulés pelliculés, comprenant l'étape de :

enrobage des granulés élémentaires sphériques selon l'une quelconque des revendications 1 à 9 avec un film.

**14.** Procédé de production de granulés élémentaires sphériques selon la revendication 7, comprenant les étapes de :

pulvérisation d'une solution aqueuse ou d'une suspension aqueuse comprenant un médicament et un composé hydrosoluble de masse moléculaire élevée sur les noyaux de semence sphériques pharmaceutiquement inertes satisfaisant aux exigences (1) à (4) décrites ci-dessus par l'utilisation d'un appareil d'enrobage pelliculaire à lit fluidisé ; et
enrobage des noyaux de semence sphériques résultants avec la couche contenant un médicament.

**15.** Procédé de production de granulés élémentaires sphériques selon la revendication 14, dans lequel l'appareil d'enrobage pelliculaire à lit fluidisé est de type à lit en jaillissement comportant, dans sa partie interne, un tube de guidage (colonne de Wurster) ou de type à lit fluidisé comportant, sur sa partie inférieure, un équipement rotatif.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO63301816 B
- JP 2000001429 A
- JP HEI7173050 B
- JP HEI10139659 B
- WO 9959544 A2
- US 5384130 A
- US 5997905 A
- EP 1238662 A2